# EUROPEAN PATENT APPLICATION

(11) **EP 4 119 180 A1**
(43) Date of publication of application: **18.01.2023**
(21) Application number: 22382669.4
(22) Date of filing: 14.07.2022
(51) Int. Cl.: A61M 16/12, A61M 16/08, A61M 16/10

(54) **VALVE FOR VENTILATION DEVICES**

(30) Priority: 16.07.2021 ES 202130676
(71) Applicant: Hospital Sant Joan de Deu, 08950 Esplugues de Llobregat Barcelona (ES)
(72) Inventor: FONT VIZCARRA, Lluís, 08950 ESPLUGUES DE LLOBREGAT (Barcelona) (ES); VALLS ESTEVE, Arnau, 08950 ESPLUGUES DE LLOBREGAT (Barcelona) (ES); ALÁEZ VASCONCELLOS, Carlos, 08950 ESPLUGUES DE LLOBREGAT (Barcelona) (ES); PONS ÒDENA, Martí, 08950 ESPLUGUES DE LLOBREGAT (Barcelona) (ES)
(74) Representative: Herrero & Asociados, S.L.

(57) **Abstract**

The valve for ventilation devices comprises an end air inlet (1), a side air inlet (2), and an air outlet (8) for connection to a ventilation device, wherein the end air inlet (1) and/or the side air inlet (2) comprises a connecting piece (3, 4) removably mounted on said end air inlet (1) and/or said side air inlet (2).

Thanks to this feature, the valve is a modular type valve, so that by changing the connection piece(s) the air flow and/or oxygen concentrations can be changed as required, without having to change the whole valve.

## Description

The present invention relates to a valve for ventilation devices, in particular a Venturi type valve for ventilation devices.

### Background to the invention

In certain ventilation devices, especially those that are provided with a turbine, Venturi valves are used to regulate the air flow and oxygen concentration.

These valves of the ventilation devices require two oxygen flow inlets: One generally located at the upper end of the valve and always located before the fenestrated cylinder, which is the one that will generate the Venturi effect, and a second one, generally located at the side of the valve and always after the fenestrated cylinder. At the bottom of the valve there will be an air outlet, where an air outlet tube is connected to the patient's mask, facial interface, or helmet type mask.

Each valve of this type is designed to provide a predetermined air flow and oxygen concentration, depending on the oxygen flow delivered through the two air inlets. The main conditioning factor in this will be the diameter of the air inlet hole of the upper inlet. If these flows and concentrations were to be changed, it would be necessary to replace the entire valve with another valve.

This has the disadvantage that a hospital needs to have a large number of valves with different characteristics, which implies a high cost, both in terms of manufacturing and in terms of transport and storage.

### Description of the invention

Therefore, an objective of the present invention is to provide a valve for ventilation devices that can be adapted to different air flow and oxygen concentration characteristics.

With the valve for ventilation devices of the invention, the aforementioned disadvantages are solved, presenting other advantages which will be described below.

The valve for ventilation devices according to the present invention comprises a main body including:
- an end air inlet,
- a side air inlet, and
- an air outlet for connection to a ventilation device,
wherein the valve also comprises:
- an end valve removably mounted on the end air inlet;
the end air inlet and/or the side air inlet comprising a connecting piece removably mounted on said end air inlet and/or side air inlet.

Thanks to this feature, the valve is a modular type valve, so that by changing the connecting piece or pieces the air flow and/or oxygen concentrations can be changed as required, without having to change the whole valve.

Advantageously, the connecting piece is housed inside a hole in the end air inlet.

According to a preferred embodiment, the connecting piece for the end air inlet comprises a base portion and a tubular portion, and preferably the base portion has a diameter that substantially coincides with the diameter of the hole of the end air inlet.

In this way, a lateral support is provided to prevent breakage of the connecting piece.

Advantageously, the valve for ventilation devices according to the present invention has a frustoconical side wall, which facilitates its manufacture by means of three-dimensional printing.

In the valve for ventilation devices according to the present invention, the air outlet advantageously comprises a smooth side surface, eliminating the usual burrs of conventional valves, improving the connection with the tubes (of standard diameter) of the ventilation devices and facilitating their manufacture, as well as improving oxygen concentrations and flow.

The valve for ventilation devices according to claim 1, also comprising a side window and a perimeter flange, preferably located at a distance from said side window, e.g., at 1 mm, which ensures the expected air flow.

### Brief description of the drawings

For a better understanding of what has been explained above, some drawings are included in which, schematically and only by way of a non-limiting example, a practical case of embodiment is shown.
Figure 1 is an elevational view of the valve for ventilation devices according to the present invention; and
Figure 2 is a perspective view of the upper part of the valve for ventilation devices according to the present invention, where the connecting piece for the end air inlet can be seen.

### Description of a preferred embodiment

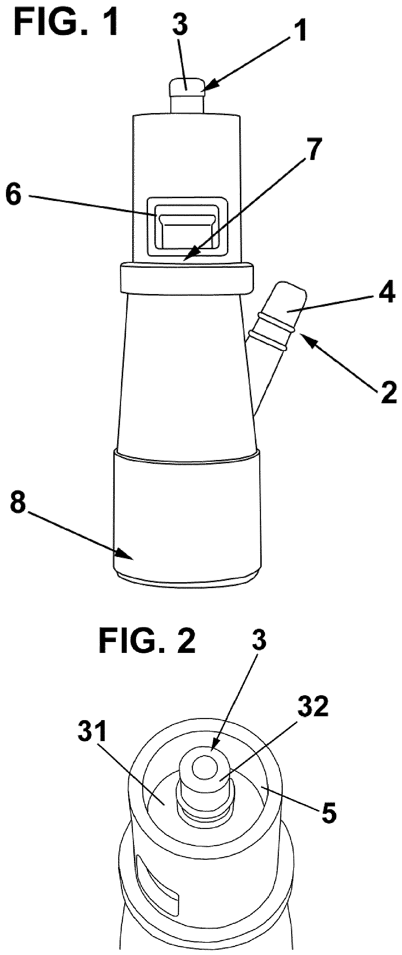

As shown in figure 1, the valve for venting devices according to the present invention is a Venturi type valve comprising an end inlet 1, located at the upper end of the valve in this figure and a side inlet 2, located at one side of the valve.

These inlets are air inlets and, according to the embodiment shown, comprise connecting pieces 3, 4 for their connection to an air inlet.

These connecting pieces 3, 4 are removably mounted on the valve body, i.e., they can be easily attached and removed to change the flow and oxygen concentration characteristics of the valve.

In this way, a modular valve is achieved, in which only these connecting parts 3, 4 need to be replaced.

It should be noted that it is not essential that the valve comprises both connecting pieces 3, 4, as it is possible to use only one connecting piece.

As can be seen in figure 2, the connecting piece 3 for the end inlet 1 comprises a base portion 31 with a circular profile and a tubular portion 32 protruding from the rest of the valve.

Said base portion 31 is housed in a hole 5 of the end inlet 1, and the diameters of the base portion 31 and the hole 5 are substantially the same, providing a side support that prevents breakage of the connecting piece 3.

The connecting piece 4 of the side inlet 2 is tubular in shape and the side wall of the valve is adapted for the entry of the connecting piece 4.

As can also be seen in figure 1, the side wall of the valve according to the present invention is frustoconical, which facilitates its manufacture by means of three-dimensional printing.

The valve according to the present invention also comprises a side window 6, which is usual in this type of valves, and a perimeter flange 7, which is located at a distance from said side window 6, for example, at 1 mm, which ensures the expected air flow.

The valve according to the present invention also comprises an air outlet 8 for its connection to a venting device, and which comprises a smooth side surface, eliminating the burrs common to conventional valves, improving connection to venting devices and facilitating their manufacture, as well as improving oxygen concentrations and flow.

The operation of the valve for ventilation devices according to the present invention is the usual operation of these Venturi type valves.

The difference is that instead of having to change the entire valve to change the flow and oxygen concentration, only one or both of the connecting pieces at the air inlets need to be changed.

Although reference has been made to a specific embodiment of the invention, it is obvious to a person skilled in the art that the valve for ventilation devices described is susceptible to numerous variations and modifications, and that all the details mentioned can be replaced by technically equivalent ones, without departing from the scope of protection defined by the appended claims.

## Claims

1. Valve for ventilation devices, comprising:
- an end air inlet (1),
- a side air inlet (2), and
- an air outlet (8) for connection to a ventilation device,
**characterized in that** the end air inlet (1) and/or the side air inlet (2) comprises a connecting piece (3, 4) removably mounted on said end air inlet (1) and/or said side air inlet (2).

2. Valve for ventilation devices according to claim 1, wherein the connecting piece (3) is housed inside a hole (5) of the end air inlet (1).

3. Valve for ventilation devices according to claim 1 or 2, wherein the connecting piece (3) for the end air inlet (1) comprises a base portion (31) and a tubular portion (32).

4. Valve for ventilation devices according to claims 2 and 3, wherein the base portion (31) has a diameter substantially coinciding with the diameter of the hole (5) of the end air inlet (1).

5. Valve for ventilation devices according to claim 1, having a frustoconical side wall.

6. Valve for ventilation devices according to claim 1, wherein the air outlet (8) comprises a smooth side surface.

7. Valve for ventilation devices according to claim 1, also comprising a side window (6) and a perimeter flange (7), which is located at a distance from said side window (6).
